Europäisches Patentamt

European Patent Office

Office européen des brevets

(11) Veröffentlichungsnummer: **0 164 575 B1**

(12) **EUROPÄISCHE PATENTSCHRIFT**

(19)

(45) Veröffentlichungstag der Patentschrift: **18.03.92**

(21) Anmeldenummer: **85105630.9**

(22) Anmeldetag: **08.05.85**

Teilanmeldung 90122957.5 eingereicht am 08/05/85.

(51) Int. Cl.⁵: **C12N 15/05**, C12N 15/82, C12N 15/87

(54) Transformation von Pflanzenerbgut.

(30) Priorität: **11.05.84 CH 2336/84**
**11.02.85 CH 606/85**
**01.04.85 CH 1398/85**

(43) Veröffentlichungstag der Anmeldung:
**18.12.85 Patentblatt 85/51**

(45) Bekanntmachung des Hinweises auf die Patenterteilung:
**18.03.92 Patentblatt 92/12**

(84) Benannte Vertragsstaaten:
**AT BE CH DE FR IT LI LU NL SE**

(56) Entgegenhaltungen:
**EP-A- 140 504**
**EP-A- 0 086 537**
**EP-A- 0 114 529**

(73) Patentinhaber: **CIBA-GEIGY AG**
**Klybeckstrasse 141**
**CH-4002 Basel(CH)**

(72) Erfinder: **Paszkowski, Jerzy, Dr.**
**Oberdorfstrasse 5**
**CH-4125 Riehen(CH)**
Erfinder: **Potrykus, Ingo, Dr.**
**Im Stigler 54**
**CH-4312 Magden(CH)**
Erfinder: **Hohn, Barbara, Dr.**
**Talmattweg 11**
**CH-4103 Bottmingen(CH)**
Erfinder: **Shillito, Raymond Douglas, Dr.**
**Kohlplatzweg 22**
**CH-4310 Rheinfelden(CH)**
Erfinder: **Hohn, Thomas, Dr.**
**Talmattweg 11**
**CH-4103 Bottmingen(CH)**
Erfinder: **Saul, Michael William, Dr.**
**Bollwerkstrasse 60**
**CH-4102 Binningen(CH)**
Erfinder: **Mandak, Vaclav**
**Riehenstrasse 5/3**
**CH-4058 Basel(CH)**

(74) Vertreter: **Zumstein, Fritz, Dr. et al**
**Bräuhausstrasse 4**
**W-8000 München 2(DE)**

## Beschreibung

Die vorliegende Erfindung betrifft ein neues Verfahren zur Transformation pflanzlichen Erbgutes unter Verwendung direktes Transformationsverfahren sowie die unmittelbaren Verfahrens produkte.

Durch die Einführung neuer genetischer Information in pflanzliches Material können Pflanzen mit neuen und/oder verbesserten Eigenschaften erzeugt werden.

Angesichts des raschen Anstiegs der Weltbevölkerung und des damit verbundenen höheren Nahrungsmittel- und Rohstoffbedarfs gehört die Steigerung des Ertrags von Nutzpflanzen sowie die vermehrte Gewinnung von pflanzlichen Inhaltsstoffen, insbesondere der Fortschritt auf dem Gebiet der Nahrungs- und Heilmittel, zu den dringlichsten Aufgaben der biologischen Forschung. In diesem Zusammenhang sind als wesentliche Aspekte beispielsweise zu nennen: eine Erhöhung der Resistenz von Nutzpflanzen gegen ungünstige Bodenverhältnisse oder Klimabedingungen, gegen Krankheiten und Schädlinge, eine gesteigerte Resistenz gegen Pflanzenschutzmittel wie Insektizide, Herbizide, Fungizide und Bakterizide, und eine günstige Veränderung des Nährstoffgehalts oder des Ernteertrags von Pflanzen. Solche wünschenswerten Effekte könnten allgemein durch Induktion oder vermehrte Bildung von Schutzstoffen, wertvollen Proteinen oder Toxinen herbeigeführt werden. Eine entsprechende Beeinflussung von pflanzlichem Erbgut kann beispielsweise dadurch erfolgen, dass man ein bestimmtes Fremdgen gezielt in Pflanzenzellen einschleust, ohne hierfür von konventionellen züchterischen Massnahmen Gebrauch zu machen.

Die Uebertragung von neuen DNS-Sequenzen in Pflanzenzellen unter Verwendung von genetisch manipulierten Pflanzenbakterien sind aus der Literatur durch einige Publikationen, wie zum Beispiel Nature, Vol. 303, 209-213 (1983); Nature, Vol. 304, 184-187 (1983); Scientific American 248(6), 50-59 (1983); EMBO-Journal 2(6), 987-995 (1983); Science 222, 476-482 (1983); Science 223, 247-248 (1984); oder Proc. Natl. Acad. Sci. USA 80, 4803-4807 (1983) bekannt geworden. Die natürlichen pflanzeninfektiösen Eigenschaften dieser Bakterien wurden dabei ausgenutzt, um neues genetisches Material in Pflanzenzellen einzuschleusen. Zu diesem Zweck wurden bisher vorzugsweise Agrobacterium tumefaciens als solches oder dessen Ti-Plasmid eingesetzt, ferner auch Cauliflower-Mosaik-Virus. EP-A 86 537 beispielsweise beschreibt ein Verfahren zur direkten Transformation pflanzlicher Protoplasten, das auf der Verwendung isolierter, vom Agrobacterium-Wirt unabhängier Ti-Plasmidvektoren beruht.

In EP-A 140 504 werden Plasmidvektoren beschrieben, die für eine Transformation von Wirtszellen Verwendung finden können. Die für eine Transformation pflanzlicher Wirtszellen vorgesehenen Vektoren enthalten Kontrollsequenzen aus den Ti-Plasmiden von Agrobacterium tumefaciens.

Im Gegensatz zu den im Stand der Technik bekannten Transformationsverfahren ermöglicht das neue, erfindungsgemässe Verfahren die direkte Übertragung eines Gens in das pflanzliche Erbgut ohne den Einsatz biologischer Vektoren. Bei diesen biologischen Vektoren handelt es sich um Pathogene, wie z.B. Agrobacterium tumefaciens oder um die für die Infektiosität wesentlichen Anteile besagter Pathogene, wie z.B. das Ti-Plasmid von Agrobacterium tumefaciens. Da das erfindungsgemässe Verfahren ohne Pathogene ausgeführt wird, entfallen somit auch die Einschränkungen, welche durch die Wirtsspezifität der Pathogene gegeben sind. Die Entwicklung der Pflanzen, an denen das neuartige Verfahren der Transformation durchgeführt wird, wird durch dieses nicht beeinträchtigt.

Die vorliegende Erfindung betrifft somit in erster Linie ein Verfahren zur direkten Transformation von Genen in pflanzliches Erbgut, dass sich im wesentlichen dadurch kennzeichnet, dass man

a) ein lineares DNS Molekül oder ein Plasmid, das ein Gen enthält, welches unter der Kontrolle von pflanzenwirksamen Expressionssignalen steht, in einem geeigneten Medium und unter Anwendung geeigneter Verfahrensmassnahmen, die die Protoplasten für die Aufnahme von DNS kompetent machen, mit pflanzlichen Protoplasten in Kontakt bringt,

b) besagtes lineares DNS-Molekül bzw. Plasmid und die Protoplasten dort für einen Zeitraum belässt, der für die Aufnahme der DNS in die Protoplasten ausreicht, wobei

c) das Gen stabil in das Pflanzengenom eingebaut sowie dort repliziert wird, mit der Massgabe, dass die einzuschleusende DNS keine für die Einschleusung oder Integration wesentlichen Anteile aus Ti-Plasmiden insbesondere aber keine Anteile aus der vir-Region oder den T-DNS Borderregionen von Ti-Plasmiden enthält.

Ebenfalls umfasst von der vorliegenden Erfindung ist ein Verfahren zur Herstellung transgener Pflanzen, die einer Regeneration aus Protoplasten zugänglich sind, das sich im wesentlichen dadurch kennzeichnet, dass

a) ein lineares DNS-Molekül oder ein Plasmid, das ein Gen enthält, welches unter der Kontrolle von pflanzenwirksamen Expressionssignalen steht, in einem geeigneten Medium und unter Anwendung geeigneter Verfahrensmassnahmen, die die Protoplasten für die Aufnahme von DNS kompetent machen, direkt in pflanzliche Protoplasten einschleust,

b) besagtes lineares DNS-Molekül bzw. Plasmid und die Protoplasten dort für einen Zeitraum belässt, der für die Aufnahme der DNS in die Protoplasten ausreicht, wobei

c) das Gen stabil in das Pflanzengenom eingebaut sowie dort repliziert wird, mit der Massgabe, dass die einzuschleusende DNS keine für die Einschleusung oder Integration wesentlichen Anteile aus Ti-Plasmiden, insbesondere aber keine Anteile aus der vir-Region oder den T-DNS Borderregionen von Ti-Plasmiden enthält, und

d) aus den zuvor transformierten Protoplasten transgene Pflanzen regeneriert, welche das eingeschleuste Gen stabil in ihr Genom eingebaut enthalten.

Neben dem erfindungsgemässen Verfahren zur Transformation pflanzlichen Erbgutes betrifft die Erfindung auch die nach diesem Verfahren unmittelbar hergestellten Produkte, insbesondere Protoplasten und aus diesen hervorgegangenes pflanzliches Material, wie beispielsweise Zellen und Gewebe, insbesondere vollständige Pflanzen, welche aus den Protoplasten regeneriert worden sind, und deren genetisch identische Nachkommenschaft.

Im Rahmen der vorliegenden Erfindung gelten folgende Definitionen:

Gen:
Strukturgen mit flankierenden Expressionssignalen

Strukturgen:
Protein-kodierende DNS-Sequenz

Expressionssignale:
Promotersignal und Terminationssignal

Pflanzenwirksames Expressionssignal:
Expressionssignal, das in Pflanzen funktioniert

Promotorsignal:
die Transkription initiierendes Signal

Terminationssignal:
die Transkription terminierendes Signal

Enhancersignal:
die Transkription verstärkendes Signal

Replikationssignal:
die DNS-Replikation ermöglichendes Signal

Integrationssignal:
DNS-Sequenz, welche die Integration des Gens in die genomische DNS fördert

Hybridgen:
aus heterologen DNS-Sequenzen, d.h., DNS-Sequenzen verschiedenen Ursprungs aufgebautes Gen, wobei es sich sowohl um natürliche als auch um synthetische DNS-Sequenzen handeln kann

Träger-DNS:
das Gen flankierende neutrale, d.h., an der Funktion des Gens nicht beteiligte DNS-Sequenz

Isoliertes Gen:
aus der originären DNS herausgetrennte DNS-Sequenz, welche ein einziges Protein kodiert

NPT-II-Gen:
Neomycin-3'-Phosphotransferase-Gen, Typ II von Transposon Tn 5 (Rothstein, S.J. und W.S. Reznikoff, Cell 23, 191-199, (1981))

Genomische DNS:
DNS des Pflanzengenoms (total oder Teil davon)

Gemäss vorliegender Erfindung wird ein neues Verfahren zur Transformation pflanzlichen Erbgutes vorgeschlagen, das dadurch gekennzeichnet ist, dass man ein Gen ohne Zuhilfenahme natürlicher pflanzeninfektiöser Systeme, insbesondere aber ohne Zuhilfenahme von für die Einschleusung oder Integration wesentlichen Anteilen aus Ti-Plasmiden direkt in Pflanzenzellen überträgt. Es handelt sich hierbei also um eine Vektor-freie, direkte Transformation. Bei dieser Vektor-freien Transformation steht das einzuführende Fremdgen unter Kontrolle von pflanzenwirksamen Expressionssignalen. Vorzugsweise wird die Vektor-freie Transformation pflanzlicher Gene ausgeführt, indem man ein einzuführendes Fremdgen und als Rezipienten fungierende pflanzliche Protoplasten (Empfänger-Protoplasten) gemeinsam in eine geeignete Lösung einbringt und darin bis zur Aufnahme des Gens in die Protoplasten belässt.

Als pflanzliche Protoplasten werden vorzugsweise jeweils solche einer einzigen Pflanzenart oder einer der Art untergeordneten systematischen Einheit verwendet.

Das Fremdgen und die Protoplasten werden zweckmässigerweise über einen Zeitraum von einigen Sekunden bis zu einigen Stunden, vorzugsweise 10 bis 60 Minuten, insbesondere 30 Minuten, in der Lösung belassen.

Das erfindungsgemässe Verfahren ist breit anwendbar. So kann man beliebige Strukturgene pflanzlicher Herkunft, wie beispielsweise das Zein-Gen (Wienand, U., et al., Mol.Gen.Genet. 182, 440-444 (1981)), tierischer Herkunft, wie beispielsweise das TPA-Gen (tissue-type plasminogen activator-Gen; Pennica, D., et al., Nature 301, 214-221, (1983)), mikrobieller Herkunft wie beispieleweise das NPT-II-Gen, oder auch synthetischer Herkunft, wie beispielsweise das Insulin-Gen (Stepien, P., et al., Gene 24, 289-297 (1983)), in das Erbgut von Pflanzen übertragen, unter der Voraussetzung, dass die Strukturgene von in Pflanzen Expression bewirkenden Expressionssignalen flankiert sind, wobei die Expressionssignale pflanzlicher, tierischer, mikrobieller oder synthetischer Herkunft sein können.

Die übertragenen Gene, bestehend aus Strukturgen und flankierenden Expressionssignalen, können dabei sowohl natürlich vorkommende Gene als auch Hybridgene sein. Bevorzugt werden im erfindungsgemässen Verfahren diejenigen Gene eingesetzt, deren Expressionssignale tierischer oder insbesondere pflanzlicher oder synthetischer Herkunft sind. Beispiele für derartige Gene sind:

a) vollständige Gene aus Pflanzen, bestehend aus dem Strukturgen mit seinen natürlichen Expressionssignalen;

b) vollsynthetische Gene, bestehend aus einem Strukturgen synthetischer Herkunft, flankiert von Expressionssignalen synthetischer Herkunft;

c) Strukturgene pflanzlicher Herkunft, flankiert von pflanzenwirksamen Expressionssignalen, wobei Strukturgen und Expressionssignale von verschiedenen Pflanzenarten stammen;

d) Strukturgene pflanzlicher Herkunft, flankiert von Expressionssignalen synthetischer Herkunft;

e) Strukturgene tierischer, mikrobieller oder synthetischer Herkunft, flankiert von Expressionssignalen pflanzlicher Herkunft; oder

f) Strukturgene tierischer oder mikrobieller Herkunft, flankiert von Expressionssignalen synthetischer Herkunft.

Ganz besonders bevorzugt sind Strukturgene bakterieller Herkunft, flankiert von Expressionssignalen pflanzlicher Herkunft, insbesonsondere solchen mit Ursprung aus Pflanzenviren. Als bei der Anwendung gemäss vorliegender Erfindung besondere geeignete Expressionssignale haben sich die Expressionssignale des Gens VI des Cauliflower-Mosaik-Virus erwiesen.

Die Hybridgene werden nach an sich bekannten mikrobiologischen Verfahren hergestellt, wobei das Leseraster der Kodierung für die von der Pflanzenzelle zu produzierenden Proteine beibehalten wird. Derartige Methoden sind bekannt und werden beispielsweise in folgenden Publikationen beschrieben: "Molecular Cloning", Maniatis, T., Fritsch, E.F. und J. Sambrook, Gold Spring Harbor Laboratory, 1982, und "Recombinant DNA Techniques", Rodriguez, R.L. und R.C. Tait, Addison-Wesley Publishing Comp., London, Amsterdam, Don Mills. Ontario, Sydney, Tokyo, 1983.

Für die Integration des Fremdgens in die genomische DNS der Pflanzenzelle ist es vorteilhaft, wenn das Gen, bestehend aus Strukturgen und pflanzenwirksamen Expressionssignalen, von neutralen DNS-Sequenzen (Träger-DNS) flankiert wird. Die Träger-DNS kann dabei aus zwei linearen DNS-Strängen bestehen, so dass die in die Pflanzenzelle einzuschleusende Konstruktion ein lineares DNS-Molekül ist. Die zur Genübertragung hergestellte DNS-Sequenz kann aber auch eine ringförmige Struktur (Plasmidstruktur) haben. Solche Plasmide bestehen aus einem DNS-Strang, in den das Fremdgen mit den Expressionssignalen integriert ist. Die Träger-DNS kann synthetischen Ursprungs sein oder durch Behandlung mit geeigneten Restriktionsenzymen aus natürlich vorkommenden DNS-Sequenzen gewonnen werden. So sind beispielsweise als Träger-DNS natürlich vorkommende Plasmide geeignet, die mit einem selektiven Restriktionsenzym geöffnet worden sind.

Ein Beispiel für ein derartiges Plasmid ist das frei erhältliche Plasmid pUC8 (beschrieben in Messing, J. und J. Vieira, Gene 19, 269-276, 1982). Weiter sind als Träger-DNS auch Bruchstücke natürlich vorkommender Plasmide verwendbar. Beispielsweise ist als Träger-DNS die Deletionsmutante für Gen VI des Cauliflower-Mosaik-Virus einsetzbar.

Die Wahrscheinlichkeit der genetischen Transformation einer Pflanzenzelle kann durch verschiedene Faktoren erhöht werden. So steigt, wie man aus Versuchen mit Hefe weiss, die Anzahl der erfolgreichen, stabilen Gentransformationen

1) mit steigender Anzahl von Kopien der neuen Gene pro Zelle,
2) bei Kombination eines Replikationssignals mit dem neuen Gen, sowie
3) bei Kombination eines Integrationssignals mit dem neuen Gen.

Somit ist das erfindungsgemässe Verfahren besonders vorteilhaft anzuwenden, wenn das übertragene Gen mit einem in Pflanzenzellen wirksamen Replikations- oder einem in Pflanzenzellen wirksamen Integrationssignal oder mit beiden Signalen kombiniert ist.

Die Expression eines Gens in einer Pflanzenzelle ist abhängig von der Transkriptionshäufigkeit des Gens in eine Messenger-RNS-Sequenz. Es ist daher ebenfalls von Vorteil, wenn das neue Gen mit einem diese Transkription verstärkenden Enhancersignal kombiniert ist. Insbesondere sind diejenigen Verfahren hervorzuheben, bei denen ein Gen übertragen wird, das mit in Pflanzenzellen wirksamen Replikations-, Integrations- und Enhancersignalen kombiniert ist.

Weiterhin ist es von grossem verfahrenstechnischem Vorteil, wenn das übertragene Gen eine selektive Markerfunktion besitzt, d.h., dass die transformierten Pflanzenzellen von den nicht-transformierten Pflanzenzellen durch eine gezielte Selektionierung getrennt werden können. Eine derartige Markerfunktion erlaubt eine rationelle Arbeitsweise dadurch, dass nur diejenigen Pflanzenzellen durch übliche mikrobiologische Massnahmen zu Kalli oder vollständigen Pflanzen regeneriert werden müssen, in deren Erbgut ein zur Expression gelangendes Gen vorhanden ist, welches Marker-spezifische Selektionierungsmassnahmen gestattet.

Während als Pflanzenzellen, die als Ausgangsmaterialien für eine Transformation in Betracht zu ziehen sind, Protoplasten, Zellkulturzellen, Zellen in Pflanzengeweben, Pollen, Pollenschläuche, Eizellen, Embryosäcke oder Zygoten und Embryonen in unterschiedlichen Entwicklungsstadien zu nennen sind, sind Protoplasten wegen der direkten Einsatzmöglichkeit ohne weitere Vorbehandlungen bevorzugt.

Die Gewinnung pflanzlicher, isolierter Protoplasten, Zellen oder Gewebe kann nach an sich bekannten Methoden oder analog dazu erfolgen.

Isolierte pflanzliche Protoplasten, welche sich auch als Ausgangsmaterial für isolierte Zellen und Gewebe eignen, können von beliebigen Teilen der Pflanze gewonnen werden, wie beispielsweise von Blättern, Keimlingen, Stengeln, Blüten, Wurzeln oder Pollen. Bevorzugt werden Blattprotoplasten verwendet. Die isolierten Protoplasten können auch aus Zellkulturen gewonnen werden. Methoden für die Isolierung von Protoplasten finden sich beispielsweise in Gamborg, O.L. und Wetter, L.R., Plant Tissue Culture Methods, 1975, 11-21.

Die Uebertragung der neuen Gene in die Pflanzenzellen erfolgt auf direktem Wege ohne Benützung eines natürlichen pflanzeninfiziösen Systems, wie eines Pflanzenbakteriums, oder eines für die Infektiosität wesentlichen Anteils davon, eines Pflanzenvirus oder Uebertragung durch Insekten oder phytopathogene Pilze. Zu diesem Zweck werden die zu transformierenden Pflanzenzellen direkt mit dem zu übertragenden Gen behandelt, indem man das Fremdgen und pflanzliche Protoplasten in eine geeignete Lösung einbringt und darin über eine Zeitspanne, welche für die Aufnahme des Fremdgens in die Protoplasten ausreicht, belässt. Durch die Kombination dieser Massnahme mit Techniken, die in der mikrobiologischen Forschung zur Genübertragung angewendet werden, wie beispielsweise Poly-L-Ornithin- oder Poly-L-Lysin-Behandlung, Liposomenfusion, DNS-Proteinkomplexierung, Ladungsänderung an der Protoplastenmembran, Fusion mit mikrobiellen Protoplasten oder Calciumphosphat-Copräzipitation, und insbesondere Polyäthylenglykolbehandlung, Hitzeschock-Methode (heat shock) und Elektroporation sowie Kombination dieser drei letztgenannten Massnahmen untereinander, lässt sich die Transformationshäufigkeit steigern.

Als Lösungen, in welche das Fremdgen und die Empfängerprotoplasten eingeführt werden, kommen vorzugsweise osmotisch stabilisierte Kulturmedien, wie sie für Protoplastenkulturen verwendet werden, in Betracht.

Es stehen bereits zahlreiche Kulturmedien zur Verfügung, die sich in einzelnen Medienkomponenten oder Gruppen solcher Komponenten unterscheiden. Alle Medien sind jedoch nach dem folgenden Prinzip aufgebaut: sie enthalten eine Gruppe anorganischer Ionen im Konzentrationsbereich von ca. 10 mg/l bis zu einigen Hundert mg/l (sogenannte Makroelemente wie beispielsweise Nitrat, Phosphat, Sulfat, Kalium, Magnesium, Eisen), eine weitere Gruppe anorganischer Ionen in Mengen von maximal einigen mg/l (die sogenannten Mikroelemente wie beispielsweise Kobalt, Zink, Kupfer, Mangan), ferner eine Reihe von Vitaminen (beispielsweise Inosit, Folsäure, Thiamin), eine Energie- und Kohlenstoffquelle wie beispielsweise Saccharose oder Glukose, sowie Wachstumsregulatoren in Form natürlicher oder synthetischer Phytohormone aus den Klassen der Auxine und Cytokinine im Konzentrationsbereich von 0,01 bis 10 mg/l. Die Kulturmedien sind zudem osmotisch stabilisiert durch Zusatz von Zuckeralkoholen (beispielsweise Mannit) oder Zucker (beispielsweise Glukose) oder Salzionen (beispielsweise $CaCl_2$) und sind auf einen pH-Wert von 5,6 bis 6,5 eingestellt.

Eine nähere Beschreibung gängiger Kulturmedien findet sich beispielsweise in Koblitz, H., Methodische Aspekte der Zell- und Gewebezüchtung bei Gramineen unter besonderer Berücksichtigung der Getreide, Kulturpflanze XXII, 1974, 93-157.

Als Technik zur Gentransformation ist besonders die "Polyäthylenglykolbehandlung" geeignet, wobei im Rahmen der vorliegenden Erfindung der Begriff "Polyäthylenglykol" nicht nur für die Substanz Polyäthylenglykol selbst steht, sondern als Oberbegriff für alle Substanzen, die ebenfalls die Protoplastenmembran modifizieren und wie sie beispielsweise auf dem Gebiet der Zellfusion zum Einsatz gelangen, zu verstehen ist. Der Begriff umfasst somit beispielsweise auch andere längerkettige, mehrwertige Alkohole, wie Polypropylenglykol (425 bis 4000 g/Mol), Polyvinylalkohol oder mehrwertige Alkohole, deren Hydroxygruppen teilweise oder vollständig veräthert sind, sowie pflanzenverträgliche, auf dem Agrargebiet gebräuchliche Detergentien, wie sie beispielsweise in folgenden Publikationen beschrieben werden:
"Mc Cutcheon's Detergents and Emulsifiers Annual"
MC Publishing Corp., Ridgewood New Jersy, 1981;
Stache, H., "Tensid-Taschenbuch",
Carl Hanser Verlag, München/Wien, 1981
Sofern Polyäthylenglykol selbst eingesetzt wird (wie in den Beispielen 1 bis 3, 5 und 7), verwendet man vorzugsweise solches mit einem Molekulargewicht zwischen 1000 und 10'000 g/Mol, insbesondere zwischen 3000 und 8000 g/Mol.

Von den vorgenannten Mitteln ist die Substanz Polyäthylenglykol selbst bevorzugt.

Mit den vorgenannten Massnahmen lässt sich bereits eine beachtliche und reproduzierbare Transformationshäufigkeit von $10^{-5}$ erzielen, welche jedoch durch geeignete Massnahmen, wie sie nachfolgend näher erläutert werden, noch wesentlich verbessert werden kann.

Bei der Polyäthylenglykolbehandlung kann man beispielsweise so vorgehen, dass man entweder eine Suspension der Protoplasten in einem Kulturmedium vorlegt und anschliessend das Gen, welches gewöhnlich als Plasmid eingesetzt wird, in einem Gemisch aus Polyäthylenglykol und Kulturmedium zusetzt, oder vorteilhafterweise Protoplasten und Gen (Plasmid) im Kulturmedium vorlegt und dann erst Polyäthylenglykol zusetzt.

Im Rahmen des erfindungsgemässen Verfahrens haben sich ferner als besonders vorteilhafte Massnahmen die Elektroporation und die Hitzeschockbehandlung erwiesen.

Bei der Elektroporation (Neumann, E. et al., The EMBO Journal 7, 841-845 (1982)) werden Protoplasten in einem Osmoticum, beispielsweise einer Mannit/Magnesium-Lösung suspendiert und die Protoplastensuspension wird in die zwischen zwei Elektroden befindliche Elektroporatorkammer eingebracht. Die Protoplasten werden nun durch Entladen eines Kondensators über die Suspensionsflüssigkeit einem elektrischen Impuls von hoher Spannung und von kurzer Dauer ausgesetzt. Hierdurch wird eine Polarisierung der Protoplastenmembran und die Oeffnung von Poren in der Membran bewirkt.

Bei der Hitzebehandlung werden Protoplasten in einem Osmoticum, beispielsweise einer Mannit/Calciumchlorid-Lösung, suspendiert und in kleinen Behältern, wie beispielsweise Zentrifugenröhrchen, erhitzt, zweckmässigerweise im Wasserbad. Die Dauer der Erhitzung richtet sich nach der gewählten Temperatur. Im allgemeinen liegen die Werte im Bereich von 40°C während einer Stunde und 80°C während einer Sekunde. Optimale Ergebnisse liefert eine Temperatur von 45°C über eine Dauer von 5 Minuten. Anschliessend wird die Suspension auf Raumtemperatur oder tiefer abgekühlt.

Es hat sich ferner gezeigt, dass die Transformationshäufigkeit durch Inaktivierung der extrazellulären Nukleasen erhöht werden kann. Eine solche Inaktivierung kann durch den Einsatz pflanzenverträglicher zweiwertiger Kationen, wie beispielsweise Magnesium oder Calcium, sowie vorzugsweise auch durch Vornahme der Transformation bei hohem pH-Wert, wobei als optimaler Bereich ein pH von 9 bis 10,5 anzusehen ist, erfolgen.

Ueberraschenderweise resultiert aus dem gezielten Einsatz dieser verschiedenen Massnahmen eine enorme Steigerung der Transformationshäufigkeit, wie sie auf dem Gebiet der Gentechnologie seit langem angestrebt wird.

Je geringer bei einer Gentransformation die Transformationshäufigkeit ist, desto schwieriger und aufwendiger ist das Auffinden der wenigen, aus den transformierten Zellen resultierenden Zellklone unter der enormen Zahl nicht transformierter Klone, und die Anwendung üblicher Screening-Methoden ist bei geringer Transformationshäufigkeit nahezu oder gänzlich unmöglich, es sei denn, dass es sich um ein Gen mit selektiver Marker-Funktion handelt (z.B. Resistenz gegen eine bestimmte Substanz). Geringe Transformationshäufigkeit erfordert also bei Genen ohne Marker-Funktion einen enormen Aufwand.

Bei Transformationen mit Genen ohne Markerfunktion lassen sich übliche Screening-Methoden zur Selektionierung transformierter Zellklone erst dann sinnvoll und erfolgreich einsetzen, wenn die Transformationshäufigkeit in der Grössenordnung von Prozenten (ca. $10^{-2}$) liegt. Wie nachstehend gezeigt wird, lässt sich die angestrebte Transformationshäufigkeit mit dem erfindungsgemässen Verfahren nunmehr erreichen. Ueberraschenderweise resultiert aus dem gezielten Einsatz verschiedener Massnahmen im Rahmen des erfindungsgemässen Verfahrens eine enorme Steigerung der bisher erreichten Transformationshäufigkeit bis auf 1 bis 2 %.

Die Zusammenführung von Fremdgen und Empfänger-Protoplasten vor den übrigen Massnahmen wie beispielsweise Polyäthylenglykolbehandlung, Elektroporation und Hitzeschockbehandlung, bewirkt gegenüber einem Vorgehen in anderer Reihenfolge eine Verbesserung der Transformationshäufigkeit um etwa eine Zehnerpotenz.

Durch Elektroporation lässt sich eine Verbesserung der Transformationshäufigkeit um das 5- bis 10-fache und durch Hitzeschockbehandlung eine solche um das Zehnfache oder mehr erreichen.

Als vorteilhaft erweist sich eine Kombination von zwei oder drei der Techniken: Polyäthylenglykolbehandlung, Hitzeschockbehandlung und Elektroporation, wobei besonders gute Ergebnisse erzielt werden, wenn diese Techniken erst nach Einbringen von Fremdgen und Protoplasten in eine Lösung angewendet werden. Bevorzugt ist die Anwendung der Hitzeschockbehandlung vor der Polyäthylenglykolbehandlung und der gegebenenfalls noch nachfolgenden Elektroporation. Im allgemeinen bringt die zusätzliche Anwendung der Elektroporation noch eine weitere Steigerung der Transformationshäufigkeit, in einigen Fällen werden die durch Hitzeschock- und Polyäthylenglykolbehandlung erzielten Resultate durch zusätzliche Elektroporation jedoch nicht mehr wesentlich verbessert.

Ebenso wie die Techniken untereinander lässt sich auch der Einsatz pflanzenverträglicher zweiwertiger Kationen und/oder die Durchführung der Transformation bei einem pH von 9 bis 10,5 sowohl mit einzelnen Techniken als auch mit Kombinationen von Techniken, insbesondere mit Polyäthylenglykolbehandlung, Hitzeschockbehandlung und Elektroporation, kombinieren. Durch die zahlreichen Variationsmöglichkeiten lässt sich das erfindungsgemässe Verfahren den jeweiligen Bedingungen ausgezeichnet anpassen.

Die Kombination von Hitzeschockbehandlung, Polyäthylenglykolbehandlung und gegebenenfalls Elektroporation im Anschluss an die bereits erfolgte Zusammenführung von Fremdgen und Empfänger-Protoplasten führt nun zu einer Transformations-häufigkeit von $10^{-2}$ bis $10^{-3}$.

Mit dem erfindungsgemässen Verfahren lässt sich also eine hohe Transformationshäufigkeit erreichen, und zwar ohne Einsatz biologischer, pflanzenspezifischer Vektoren für die Transformation, wie beispielsweise Cauliflower-Mosaik-Virus oder Agrobacterium oder dessen Ti-Plasmid.

Eine vorteilhafte Methode besteht beispielsweise darin, dass man Protoplasten in eine Mannit-Lösung überführt und die so erhaltene Protoplastensuspension mit der wässrigen Genlösung vermischt. Anschliessend werden die Protoplasten in dieser Mischung 5 Minuten lang bei 45°C inkubiert und dann innerhalb 10 Sekunden auf 0°C abgekühlt. Anschliessend wird Polyäthylenglykol (MW 3000 bis 8000) zugegeben, bis eine Konzentration von 1 bis 25 %, bevorzugt von etwa 8 %, erreicht ist. Nach sorgfältiger Durchmischung erfolgt die Behandlung im Elektroporator. Anschliessend wird die Protoplastensuspension mit Kulturmedium verdünnt und die Protoplasten werden in Kultur genommen.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Bromeliaceae, Rubiaceae, Theaceae, Musaceae oder Gramineae und der Ordnung Leguminosae und hier vor allem der Familie Papilionaceae. Bevorzugt sind Vertreter der Familien Solanaceae, Cruciferae und Gramineae.

Besonders erwähnenswert sind Pflanzen der Gattungen Nicotiana, Petunia, Hyoscyamus, Brassica und Lolium, wie beispielsweise Nicotiana tabacum, Nicotiana plumbaginifolia, Petunia hybrida, Hyoscyamus muticus, Brassica napus, Brassica rapa und Lolium multiflorum.

Die Kulturpflanzen mit grossen Ernteerträgen wie Mais, Reis, Weizen, Gerste, Roggen, Hafer oder Hirse sind in erster Linie Objekt der Anstrengungen auf dem Gebiet der Transformation von Pflanzenzellen.

Alle Pflanzen, die durch Regeneration aus Protoplasten erzeugt werden können, lassen sich auch unter Anwendung des erfindungsgemässen Verfahrens transformieren. Vertreter der Familie Gramineae (Gräser), welche auch Getreide umfasst, konnten bisher nicht genetisch manipuliert werden. Es wurde nunmehr nachgewiesen, dass mit der vorstehend beschriebenen Methode der direkten Gentransformation auch Gramineenzellen, also auch Getreidezellen, genetisch transformiert werden können. In gleicher Weise ist eine Transformation der Kulturpflanzen der Gattungen Solanum, Nicotiana, Brassica, Beta, Pisum, Phaseolus, Glycine, Helianthus, Allium, Weizen, Gerste, Hafer, Setaria, Raps, Reis, Cydonia, Pyrus, Malus, Rubus, Fragaria, Prunus, Arachis, Secale, Panicum, Saccharum, Coffea, Camellia, Musa, Ananas, Vitis oder Citrus möglich und erwünscht, wenn auch die Gesamterträge und -anbauflächen hierfür weltweit geringer sind.

Der Nachweis transformierter Gene kann auf an sich bekannte Weise erfolgen, beispielsweise durch Kreuzungsanalysen und molekularbiologische Untersuchungen, zu denen besonders die "Southern blot"-Analyse und Enzymaktivitätstests gehören.

Die "Southern blot"-Analyse kann beispielsweise folgendermassen durchgeführt werden: Die den transformierten Zellen oder Protoplasten entnommene DNS wird nach Behandlung mit Restriktionsenzymen einer Elektrophorese in 1 %-igem Agarose-Gel unterworfen, auf eine Nitrozellulosemembran (Southern, E.M., J.Mol.Biol. 98, 503-517 (1975)) transferiert und mit der nachzuweisenden DNS, welche einer Nick-Translation (Rigby, W.J., Dieckmann, M., Rhodes, C.und P.Berg, J.Mol. Biol. 113, 237-251, (1971)) unterworfen wurde (DNS-spezifische Aktivität von $5 \times 10^8$ bis $10 \times 10^8$ c.p.m./$\mu$g) hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNS wird durch Schwärzung eines Röntgenfilms während 24 bis 48 Stunden sichtbar gemacht.

Eine Untersuchung auf Enzymaktivität lässt sich - näher erläutert am Test auf Aminoglykosidphosphotransferase (Enzym für Kanamycinspezifische Phosphorylierung) - beispielsweise folgendermassen durchführen: Kallus- oder Blattstücke (100 bis 200 mg) werden in 20 $\mu$l Extraktionspuffer in einem Eppendorf-Zentrifugenröhrchen homogenisiert. Der Puffer ist eine Modifikation des von Herrera-Estrella, L., DeBlock, M., Messens, E., Hernalsteens, J.-P., Van Montagu, M. und J. Schell, EMBO J. 2, 987-995 (1983) verwendeten Puffers, in welchem Albumin aus Rinderblut weggelassen und 0,1 M Sucrose hinzugefügt worden sind. Die Extrakte werden 5 Minuten lang bei 12'000 g zentrifugiert und die überstehende Phase mit Bromphenolblau bis zu einer Endkonzentration von 0,004 % versetzt. Durch Elektrophorese in einem 10 %-igen, nicht denaturierenden Polyacrylamid-Gel werden die Proteine aus 35 $\mu$l der überstehenden Phase separiert. Das Gel wird mit einem Kanamycin und $\gamma$-$^{32}$P-markiertes ATP enthaltenden Agarose-Gel überschichtet, inkubiert, und die phophorylierten Reaktionsprodukte werden auf Whatman-p81-Phosphozellulose-Papier übertragen. Das Papier wird sechsmal mit deionisiertem Wasser von 90°C gewaschen und dann autoradiografiert.

Die nachfolgenden Beispiele dienen der näheren Illustration der vorliegenden Erfindung, ohne diese jedoch zu begrenzen. Sie beschreiben die Konstruktion eines Hybridgens und dessen Einbau in Träger-DNS-Sequenzen mit cyclischem Charakter, die Uebertragung dieses Hybridgens in Pflanzenzellen, die Selektionierung der transformierten Pflanzenzellen und die Regeneration von vollständigen Pflanzen aus den transformierten Pflanzenzellen sowie deren kreuzungsgenetische und molekularbiologische Analyse.

In den Beispielen wird das erfindungsgemässe Verfahren wie folgt illustriert:

1) durch Transformation von Tabakpflanzen mittels Uebertragung des NPT-II-Gens, in der Weise, dass man das NPT-II-Gen mit Promotor- und Tertinationssignalen des CaMV-Gens VI versieht, dieses in das pUC8-Plasmid einbaut und das erhaltene chimäre Plasmid durch Polyäthylenglykol-Behandlung in isolierte Tabakprotoplasten überträgt,

2) durch Transformation von Pflanzen der Gattung Brassica mittels Uebertragung des NPT-II-Gens, in der Weise, dass man das NPT-II-Gen mit Promotor- und Terminationssignalen des CaMV-Gens VI versieht, diese Konstruktion anstelle des CaMV-Gens VI in das CaMV-Genom einbaut und das erhaltene chimäre Plasmid durch Polyäthylenglykol-Behandlung in isolierte Bressicaprotoplasten überträgt, und

3) durch Transformation von Pflanzen der Gattung Lolium mittels Uebertragung des NPT-II-Gens, in der Weise, dass man das NPT-II-Gen mit Promotor- und Terminationssignalen des CaMV-Gens VI versieht, dieses in das pUC8-Plasmid einbaut und das erhaltene chimäre Plasmid durch Polyäthylenglykol-Behandlung in isolierte Loliumprotoplasten überträgt.

Ferner wird auch die vorteilhafte Beeinflussung der Transformation durch Hitzeschockbehandlung und Elektroporation sowie der kombinierten Methode von Hitzeschockbehandlung, Polyäthlenglykolbehandlung und Elektroporation nach erfolgter Zusammenführung von Protoplasten und NPT-II-Gen durch Beispiele veranschaulicht.

Beispiel 1: Transformation von Zellen von Nicotiana tabacum c.v. Petit Havana SRI durch Uebertragung des NPT-II-Gens

a) Konstruktion des Plasmids pABDI

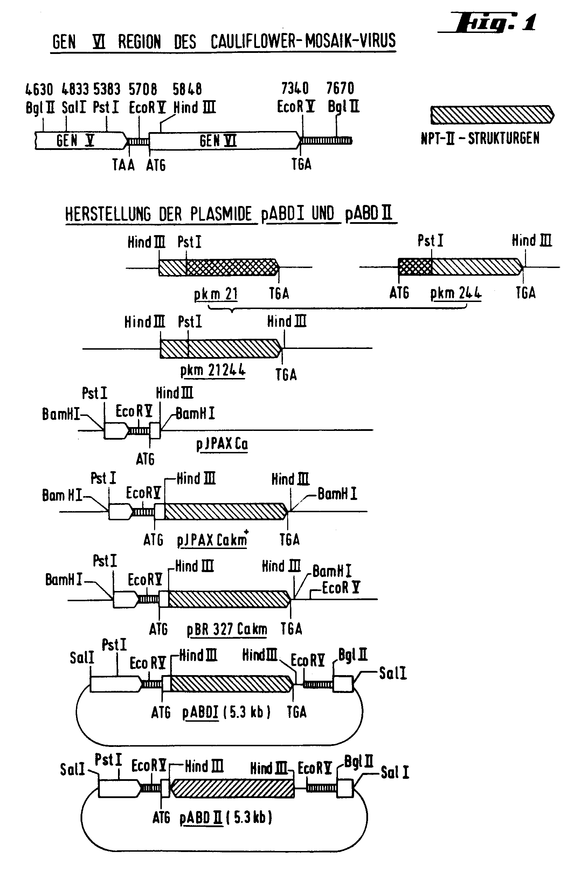

Man zerschneidet die frei zugänglichen Plasmide pkm 21 und pkm 244 (Beck, E., et al., Gene 19, 327-336 (1982)) mit der Restriktions-Endonuclease PstI. Die Fragmente der Plasmide, welche für die Rekombination verwendet werden, werden durch Elektrophorese in 0,8%igem Agarose-Gel gereinigt. Das aus der Verbindung der Bruchstücke erhaltene Plasmid pkm 21244 enthält eine Kombination der 5'-und 3'-Bal 31 - Deletionen des NPT-II-Gens, wie von Beck et al in Gene 19, 327-336 (1982) beschrieben. Um das Promotorsignal des Cauliflower-Mosaik-Virus mit dem HindIII-Fragment des Plasmids pkm 21244 zu verbinden, wird das Kupplungsplasmid pJPAX konstruiert. Das Kupplungsplasmid pJPAX wird aus den Plasmiden pUC8 und pUC9 (Messing, J. and J. Vieira, Gene 19, 269-276 (1982)) erhalten. 10 Basenpaare von der Kupplungssequenz des Plasmids pUC9 werden durch Zerschneiden bei der HindIII- und der SalI-Position und nachfolgendem Auffüllen der haftfähigen Enden mit den Polymerase-I-Klenow-Fragment (Jacobsen, H., et el., Eur. J. Biochem. 45, 623, (1974)) und Verbinden der Polynukleotidkette, wodurch die HindIII-Position wieder hergestellt wird, herausgetrennt. Ein synthetisches Kupplungsglied von 8 Basenpaaren (XhoI) wird an der SmaI-Position dieser abgetrennten Kupplungssequenz eingefügt. Die Rekombination der geeigneten XorI- und HindIII-Fragmente des Plasmids pUC8 und des modifizierten Plasmids pUC9 ergibt das Plasmid pJPAX mit einer teilweise asymmetrischen Kupplungssequenz mit den aufeinander folgenden Restriktionsstellen: EcoRI, SmaI, BamHI, SalI, PstI, HindIII, BamHI, XhoI und EcoRI. Die Verbindung des 5'-Expressionssignals des Cauliflower-Mosaik-Virus-Gens VI und des HindIII-Fragments des NPT-II-Gens wird beim Plasmid pJPAX durch Einfügung der Promotorregion des Cauliflower-Mosaik-Virus-Gens VI zwischen die PstI- und die Hind III-Position bewirkt. Das so erhaltene Plasmid wird an der einzigen HindIII-Position aufgeschnitten und das HindIII-Fragment des Plasmids pkm 21244 wird in diese Schnittstelle in beiden Orientierungsrichtungen eingebaut, wobei man die Plasmide pJPAX Cakm$^+$ und pJPAX Cakm$^-$ erhält. Um eine EcoRV-Sequenz in der Nähe des 3'Terminationssignals des NPT-II-Hybridgens zu schaffen, wird ein BamHI-Fragment des Plasmids pJPAX Cakm$^+$ in die BamHI-Position des Plasmids pBR 327 (Soberon, X. et al., Gene 9, 287-305 (1980)) eingebaut. Man erhält das Plasmid pBR 327 Cakm. Das EcoRV-Fragment des Plasmids pBR 327 Cakm, das die neue DNS-Konstruktion enthält, wird verwendet, um die EcoRV-Region des Cauliflower-Mosaik-Virus-Gens VI zu ersetzen, welches an der SalI-Position im Plasmid pUC8 kloniert ist, wodurch man die Proteinkodierende DNS-Sequenz des NPT-II-Gens unter die Kontrolle der 5'- und 3'-Expressionssignale des Cauliflower-Mosaik-Gens VI stellt. Die so hergestellten Plasmide tragen die Bezeichnung pABDI und pABDII (vgl. Figur 1).

b) Transformation von Protoplasten von Nicotiana tabacum c.v. Petit Havanna SRI durch Uebertragung den NPT-II-Gens als Teil des Plasmids pABDI mittels PEG-Behandlung.

In 1 ml K$_3$-Medium [siehe Z. Pflanzenphysiologie 78, 453-455 (1976); Mutation Research 81 - (1981) 165-175], enthaltend 0,1 mg/l 2,4-Dichlorphenoxyessigsäure, 1,0 mg/l 1-Naphthylessigsäure und 0,2 mg/l 6-Benzylaminopurin werden in einer Populationsdichte von 2•10$^6$ pro ml Tabakprotoplasten suspendiert, die zuvor aus einer Enzymsuspension durch Flotation auf 0,6 molarer Sucrose bei pH 5,8 und anschliessender Sedimentation (100 g für 5 Minuten) in 0,17 molarem Calcium-chlorid bei pH 5,8 gewonnen worden sind. Zu dieser Suspension werden nacheinander 0,5 ml 40%iges Polyäthylenglykol MG 6000 (PEG) in modifiziertem (nach dem Autoklavieren erneut auf pH 5,8 eingestelltem) F-Medium (Nature 296, 72-14 (1982)) und 65 µl einer wässrigen Lösung, enthaltend 15 µg des Plasmids pABDI und 50 µg Kalbsthymus-DNS zugesetzt. Diese Mischung wird unter gelegentlichem Umschwenken für 30 Minuten bei 26°C inkubiert und anschliessend stufenweise mit F-Medium verdünnt. Die Protoplasten werden durch Zentrifigieren (5 Minuten bei 100 g) abgetrennt und in 30 ml frischem K$_3$-Medium resuspendiert. Die weitere Inkubation erfolgt in 10 ml-Portionen in Petri-Schalen von 10 ca Durchmesser bei 24°C und Dunkelheit, wobei die Populationsdichte 6,3•10$^4$ Protoplasten pro ml beträgt. Nach 3 Tagen wird das Kulturmedium pro Schale mit 0,3 Volumenteilen frischem K$_3$-Medium verdünnt und für weitere 4 Tage bei 24°C und 3000 lux künstlicher Beleuchtung inkubiert. Nach insgesamt 7 Tagen werden die aus den Protoplasten entwickelten Klone in mit 1 % Agarose verfestigtem, 50 mg/l Kanamycin enthaltendem Nährmedium eingebettet und nach der "bead type"-Kulturmethode [Plant Cell Reports, 2, 244-247 (1983)] bei 24°C unter Dunkelheit kultiviert. Das Nährmedium wird alle 5 Tage durch frische gleichartige Nährlösung ersetzt.

c) Regeneration Kanamycin-resistenter Tabakpflanzen

Nach 3 bis 4 Wochen fortgesetzter Kultivierung in Kanamycin-haltigem Nährmedium werden die resistenten Kalli von 2 bis 3 mm Durchmesser auf mit Agar verfestigtem LS-Kulturmedium [Physiol. Plant 18, 100-127 (1965)], enthaltend 0,05 mg/l 2,4-Dichlorphenoxyessigsäure, 2 mg/l 1-Naphthylessigsäure, 0,1 mg/l 6-Benzylaminopurin, 0,1 mg/l Kinetin und 75 mg/l Kanamycin, verpflanzt. Durch Sprossinduktion auf LS-Medium, enthaltend 150 mg/l Kanamycin und 0,2 mg/l 6-Benzylaminopurin, und anschliessender Wurzelbildung auf T-Medium [Science 163, 85-87 (1969)] erhält man Kanamycin-resistente Nicotiana tabacum-Pflanzen der Sorte Petit Havanna SRI.

d) Nachweis des NPT-II-Gens im Pflanzenerbgut

0,5 g Kallus der transformierten Zellkulturen oder Blattgewebe der daraus regenerierten Pflanzen werden bei 0°C in 15 %-iger Saccharoselösung, enthaltend 50 mMol/l Aethylendiamin-N,N,N',N'-tetraessigsäure, EDTA), 0,25 Mol/l Natriumchlorid und 50 mMol/l α,α,α-Tris-(hydroxymethyl)-methylamin-Hydrochlorid (TRIS-HCl) bei pH 8,0 homogenisiert. Durch Zentrifugieren des Homogenisats für 5 Minuten bei 1000 g trennt man grob die Zellkerne ab. Die Zellkerne werden in 15 %-iger Saccharoselösung, enthaltend 50 mMol/l EDTA und 50 mMol/l TRIS-HCl, bei pH 8,0 resuspendiert, mit Natrium-dodecylsulfat bis zu einer Endkonzentration von 0,2 % versetzt und für 10 Minuten auf 70°C erhitzt. Nach Abkühlung auf 20-25°C wird der Mischung Kaliumacetat bis zu einer Konzentration von 0,5 Mol/l zugesetzt. Dieses Gemisch wird für 1 Stunde bei 0°C inkubiert. Der ausgefallene Niederschlag wird durch Zentrifugieren (15 Minuten bei 4°C in einer Mikrozentrifuge) zentrifugiert. Aus der überstehenden Flüssigkeit wird durch Versetzen mit dem 2,5-fachen Volumen Aethanol bei 20-25°C die DNS ausgefällt. Die abgetrennte DNS wird in einer Lösung von 10 mMol TRIS-HCl, enthaltend 10 µg/ml Ribonuclease A, gelöst, für 10 Minuten bei 37°C inkubiert, mit Proteinase K bis zu einer Konzentration von 250 µg/ml versetzt und für eine weitere Stunde bei 37°C inkubiert. Die Proteinase K wird durch Phenol- und Chloroform/Isoamylalkohol-Extraktionen enfernt. Aus der wässrigen Phase wird die DNS durch Zusatz von 0,6 Volumenteilen 0,6 molarer isopropanolischer Natriumacetat-Lösung ausgefällt und in 50 µl einer Lösung, enthaltend 10 mMol/l TRIS-HCl und 5 mMol/l EDTA, bei pH 7,5 gelöst. Durch diese Präparation erhält man DNS-Sequenzen, die vorwiegend mehr als 50'000 Basenpaare enthalten. Restriktion dieser DNA mit EcoRV-Endonuclease, Hybridisierung der Fragmente mit radioaktiv markierten HindIII-Bruchstükken des NPT-II-Gens und Vergleich mit dem Plasmid pABDI zeigt in einer "Southern Blot"-Analyse die Anwesenheit des NPT-II-Gens in der Zellkern-DNS der transformierten Nicotiana tabacum-Zellen.

e) Nachweis der Uebertragung des transformierten Gens auf die sexuellen Nachkommenschaften sowie seiner Vererbung als normales Pflanzengen

Umfangreiche genetische Kreuzungsanalysen und ausführliche molekularbiologische Studien (beispielsweise "Southern blot"-Analyse der DNS des Pflanzengenoms; Untersuchung der Enzymaktivität der Aminoglykosidphosphotransferase, d.h., dem Enzym für die Kanamycinspezifische Phosphorylierung) mit den genetisch veränderten Pflanzen (erste Generation und Nachkommenschaften) haben folgende, Ergebnisse erbracht:

1. Das bakterielle Gen ist stabil in das Pflanzengenom eingebaut;

2. Es wird in der Regel unverändert und regelmässig auf Kreuzungsnachkommenschaftenübertragen;

3. Sein Erbgang entspricht dem eines natürlichen, einfachen, dominanten Pflanzengens;

4. Die molekulare Analyse mittels DNS-Hybridisierung und Enzymtest bestätigt die Ergebnisse der genetischen Kreuzungsanalyse;

5. Die genetisch veränderten Pflanzen haben ihren normalen, natürlichen Phänotyp während der Behandlung beibehalten; es sind also keine unerwünschten Veränderungen festgestellt worden.

Diese Ergebnisse zeigen, dass mit dem erfindungsgemässen Verfahren des direkten Gentransfers in Protoplasten der beste Weg für die gezielte genetische Veränderung von pflanzlichem Material aufgefunden worden ist. Die genetische Veränderung ist stabil, und unerwünschte Aenderungen des Genotyps der Pflanze treten nicht auf.

Entsprechende Resultate werden bei Durchführung der im vorstehenden Beispiel beschriebenen Transformation auch mit Nicotiana plumbaginifolia, Petunia hybrida, Hyoscyamus muticus und Brassica napus erhalten.

Beispiel 2: Transformation von Zellen von Brassica rapa c.v. Just Right durch Uebertragung des NPT-II-Gens

a) Konstruktion den Plasmids pCaMV6km

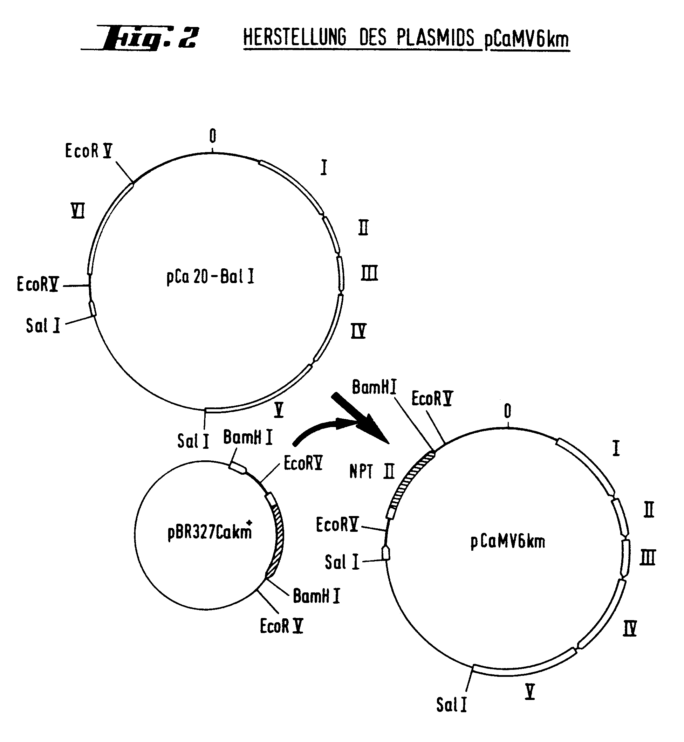

Das unter Beispiel 1a) beschriebene Plasmid pBR 327 Cakm$^+$ wird mit Restriktionsendonuclease EcoRV geschnitten und das EcoRV-Restriktionsfragment mit dem Kanamycin-Resistenz-Gen (NPT-II) gegen das das Gen VI von Cauliflower-Mosaik-Virus enthaltende EcoRV-Fragment des Plasmids pCa20-Bal 1 ausgetauscht. Man erhält so das Plasmid pCaMV6km (Figur 2). Bei dem Plasmid Ca20-Bal 1 handelt es sich um ein chimäres CaMV-Plasmid, welches von der natürlichen Deletionsmutante CM4-184 abstammt. In diesem Plasmid fehlt mit Ausnahme der ersten fünf Codons und dem Translations-Stopsignal TGA die gesamte Region II. Ein XhoI-Kupplungsfragment wurde unmittelbar vor dem Stop-Codon in der Region II eingefügt.

b) Transformation von Protoplasten von Brassica rapa c.v. Just Right durch Uebertragung des NPT-II-Gens als Teil des Plasmids pCaMV6km mittels PEG-Behandlung

Brassica rapa-Protoplasten werden mit einem geeigneten Osmotikum gewaschen und in einer Populationsdichte von 5 • 10$^6$ pro ml in einem Kulturmedium, hergestellt gemäss Protoplasts 83, Poster Proceedings Experientia Supplementum, Birkhäuser Verlag, Basel, Vol. 45 (1983), 44-45, suspendiert. 40 %-iges Polyäthylenglykol MG 6000 (PEG) gelöst in modifiziertem F-Medium (pH 5,8) (vgl. Beispiel 1b) werden mit der Protoplasten-Suspension bis zu einer Endkonzentration von 13 % PEG vermischt. Dieser Mischung wird umgehend eine Lösung von 10 μg mit Endonuclease SalI verdautem Plasmid pCaMV6km und 50 μg Kalbsthymus-DNS in 60 μl Wasser zugesetzt. Unter gelegentlichem Unschwenken wird die Mischung für 30 Minuten bei 20-25°C inkubiert. Anschliessend werden im Abstand von jeweils 5 Minuten dreimal je 2 ml modifiziertes F-Medium (insgesamt 6 ml) und zweimal je 2 ml Kulturmedium (insgesamt 4 ml) zugesetzt. Die so erhaltene Protoplastensuspension wird in Petrischalen von 10 cm Durchmesser überführt und mit weiteres Kulturmedium auf ein Gesamtvolumen von 20 ml aufgefüllt. Diese Protoplastensuspensionen werden bei Dunkelheit für 45 Minuten bei 26°C inkubiert. Die Protoplasten werden durch Sedimentation für 5 Minuten bei 100 g abgetrennt, in ein zunächst flüssiges, dann gelierendes Agarose-Gel-Kulturmedium aufgenommen und nach der "bead type"-Kulturtechnik [Plant Cell Reports 2, 244-247 (1983)] kultiviert. Nach 4 Tagen, im Entwicklungsstadium der ersten Zellteilung, wird den Kulturen Kanamycin in einer Konzentration von 50 mg/l zugesetzt. Das die Agarosesegmente umgebende flüssige Kulturmedium wird alle 4 Tage durch frische, Kanamycinhaltige Nährlösung ersetzt. Nach 4 Wochen isoliert man die Kanamycin-resistenten Klone. Diese werden weitergezüchtet, indes sie wöchentlich mit Kanamycin-haltiger Nährlösung (50 mg/l) versorgt werden.

c) Nachweis des NPT-II-Gens im Pflanzenerbgut

Durch Isolation der Zellkern-DNS und deren Restriktion und Hybridisierung der DNS-Bruchstücke kann analog zum Beispiel 1d) das Vorhandensein des NPT-II-Gens im Zellkern der transformierten Brassica rapa-Zellen nachgewiesen werden.

Beispiel 3: Transformation von Gramineenprotoplasten der Spezies Lolium multiflcrum

Protoplasten von Lolium multiflorum (Italienisches Raygras) werden in einer Konzentration von 2•10$^6$ pro ml in 1 ml 0,4 molarem Mannit, pH 5.8 aufgenommen. Zu dieser Suspension werden nacheinander 0,5 ml 40 %-iges Polyäthylenglykol MG 6000 (PEG) in modifiziertem (pH 5.8) F-Medium (Nature 296, 72-74 (1982)), und 65 µl einer wässrigen Lösung, enthaltend 15 µg des Plasmids pABDI und 50 µg Kalbsthymus-DNS zugesetzt. Diese Mischung wird unter gelegentlichem Umschwenken für 30 Minuten bei 26°C inkubiert und anschliessend stufenweise mit F-Medium verdünnt, wie in Nature 296 (1982) 72-74 beschrieben. Die Protoplasten werden durch Zentrifugieren (5 Minuten bei 100 g) abgetrennt und in 4 ml CC-Kulturmedium [Potrykus, Harms, Lörz, Callus formation from cell culture protoplasts of corn (Zea mays L.), Theor. Appl. Genet. 54, 209-214 (1979)] aufgenommen und bei 24°C in Dunkelheit inkubiert. Nach 14 Tagen werden die heranwachsenden Zellkulturen in das gleiche Kulturmedium, jedoch mit dem Antibiotikum G-418 (kommerziell erhältlich; GIBCO EUROPE Produktekatalog, Katalog-Nr. 0661811) übertragen. G-418 ist bei einer Konzentration von 25 mg/Liter toxisch für Loliumzellen und gestattet ausschliesslich die Weiterentwicklung von Zellen, welche das bakterielle Gen für Kanamycinresistenz aufgenommen haben. G-418 ist ein Kanamycin-Analoges mit wesentlich besserer Wirksamkeit in Gramineenzellen, als Kanamycin selbst. Resistente Zellkolonien werden auf Agarmedium (gleiches Medium wie oben, 25 ml/l G-418, ohne Osmotikum) übertragen und nach Erreichen einer Grösse von mehreren Gramm Frischgewicht pro Zellkolonie, auf das Vorliegen des bakteriellen Gens und auf biologische Aktivität des Gens analysiert. Ersteres erfolgt durch Hybridisierung einer radioaktiv markierten DNA-Probe des Gens mit DNA, die aus der Zellkultur isoliert wurde; letzteres geschieht durch Nachweis der Enzymaktivität durch Phosphorylierung von Kanamycin mit radioaktivem ATP. Beide molekularen Nachweisverfahren erbrachten den eindeutigen Beweis für die genetische Transformation der Zellkolonien, die auf G-418 selektioniert worden waren. Die Versuche stellen den erstmaligen Nachweis der genetischen Transformation von Gramineenprotoplasten dar, und beweisen überdies, dass mit dem beschriebenen Verfahren Gräserprotoplasten prinzipiell genetisch manipuliert werden können. Somit ist auch die Möglichkeit der genetischen Manipulation von Kulturgräsern, wie z.B von Getreide, eröffnet.

**Beispiel 4: Transformation von Zellkultur-Zellen von Nicotiana tabacum durch Uebertragung des NPT-II-Gens unter Anwendung der Elektroporation**

Von 50 ml einer log-Phasen-Suspensionskultur der Nitratreduktase-Mangel-Variante von Nicotiana tabacum, Zellstamm nia-115 (Müller, A.J., und R. Grafe, Mol.Gen.Genet. 161, 67-76 (1978)) werden die Protoplasten durch Sedimentation hergestellt, in 20 ml Enzymlösung [2 % Cellulase Onozuka R-10, 1 % Mazerozym R-10 und 0,5 % Driselase (Chemische Fabrik Schweizerhalle, Basel) in Waschlösung (0,3 M Mannit, 0,04 M Calciumchlorid und 0,5 % 2-(N-Morpholino)-äthansulfonsäure); mit KOH auf pH 5,6 eingestellt] resuspendiert und während drei Stunden auf einem Rotationsschüttelapparat bei 24°C inkubiert. Dann werden die Protoplasten durch ein Sieb von 100 µm Maschenweite filtriert, wodurch die unverdauten Gewebereste zurückgehalten werden, mit einer volumengleichen Menge 0,6 M Sucrose versetzt und bei 100 g während 10 Minuten zentrifugiert. Die an der Oberfläche flottierenden Protoplasten werden gesammelt und dreimal durch Sedimentation in der Waschlösung gewaschen.

Die Transformation erfolgt unter Anwendung der Elektroporation. Die Kammer vom Dialog[R] "Porator" (Dialog GmbH, Harffstr. 34, 4000 Düsseldorf, Deutschland-West) wird durch Waschen mit 70 %-igem Aethanol und anschliessendem Waschen mit 100 %-igem Aethanol sterilisiert und zum Trocknen einem sterilen Luftstrom aus einem Gebläse mit laminarer Luftströmung ausgesetzt. Die Protoplasten werden in einer Konzentration von $1 \times 10^6$/ml in 0,4 M Mannitol-Lösung suspendiert, mit Magnesiumchlorid auf einen Widerstand von 1,4 kOhm einjustiert und mit pABDI-DNS in einer Konzentration von 10 µg/ml versetzt. Portionen von jeweils 0,38 ml dieser Protoplastensuspension werden 3mal in Abständen von jeweils 10 Sekunden einem Impuls von 1000 Volt oder einen Impuls von 2000 Volt ausgesetzt. Die Protoplasten werden anschliessend in einer Konzentration von $1 \times 10^5$/ml in 3 ml AA-CH-Medium (AA-Medium nach Glimelius, K. et al., Physiol. Plant. 44, 273-277 (1978), modifiziert durch Erhöhung des Inosit-Gehaltes auf 100 mg/l und des Saccharose-Gehaltes auf 34 g/l sowie Zusatz von 0,05 ml/l 2-(3-Methyl-2-butenyl)-adenin), welches durch einen Gehalt an 0,6 % Agarose (Sea Plaque, FMC Corp., Marine Colloids Division, P.O. Box 308, Rockland, Maine 04841, USA) in den festen Zustand übergeht, kultiviert. Nach einer Woche wird die die Protoplasten enthaltende Agaroseschicht in 30 ml flüssiges AA-CH-Medium, welches 50 mg/l Kanamycin enthält, überführt. Nach drei Wochen, in denen wöchentlich die Hälfte des flüssigen Mediums durch frisches Medium gleicher Zusammensetzung ersetzt wird, lassen sich die transformierten Zellkolonien optisch wahrnehmen. Diese Zellkolonien werden zur Weiterkultivierung und Untersuchung 4 Wochen, nachdem die Ueberführung in das Kanamycin enthaltende Medium erfolgt ist, auf AA-Medium (Glimelius, K., et al., Physiol. Plant. 44, 273-277 (1978); 0,8 % Agar), welches 50 mg/l Kanamycin enthält, überführt. Die Bestätigung der erfolgreichen Transformation erfolgt durch DNS-Hybridisierung und Prüfung der Enzymaktivität der Aminoglykosidphosphotransferase.

Analoge Versuche mit Protoplasten von Brassica rapa und Lolium multiflorum führen ebenfalls zu erfolgreichen Transformationen.

Beispiel 5: Transformation von Zellen von Nicotiana tabacum durch Uebertragung des NPT-II-Gens unter Anwendung der Elektroporation

Die Vorbereitung der Elektroporatorkammer erfolgt wie im Beispiel 4 beschrieben und die der Protoplasten wie im Beispiel 1 beschrieben.

Zur Transformation werden Protoplasten von Nicotiana tabacum in einer Konzentration von 1,6 x $10^6$/ml in Mannit-Lösung (0,4 M, gepuffert mit 0,5 Gew. %/Vol. 2-(N-Morpholino)-äthansulfonsäure; pH 5,6) resuspendiert. Der Widerstand der Protoplastensuspension wird in der Poratorkammer (0,38 ml) gemessen und mit Magnesiumchlorid-Lösung (0,3 M) auf 1 bis 1,2 kOhm eingestellt. Portionen von je 0,5 ml werden in verschliessbare Plastikröhrchen (5 ml Volumen) gegeben und pro Röhrchen zunächst 40 $\mu$l Wasser, welches 8 $\mu$g pABDI (in die lineare Form überführt mit SmaI) und 20 $\mu$g Kalbsthymus-DNS enthält, und anschliessend 0,25 ml Poläthylenglykol-Lösung (24 Gew. %/Vol in 0,4 M Mannitol) zugesetzt. 9 Minuten nach Zusatz der DNS werden Portionen von jeweils 0,38 ml in die Impulskammer eingefüllt und 10 Minuten nach der DNS-Zugabe wird die in der Kammer befindliche Protoplastensuspension 3 Impulsen (1000-2000 Volt) in Intervallen von jeweils 10 Sekunden ausgesetzt. Die so behandelten Portionen werden in Petrischalen (Durchmesser 6 cm) gegeben und 10 Minuten lang bei 20°C gehalten. Zu jeder Petrischale werden dann 3 ml $K_3$-Medium mit einem Gehalt an 0,7 Gew. %/Vol Sea-Plaque-Agarose gegeben und der Inhalt der Schale wird gut vermischt. Nach dem Erstarren des Schaleninhalts werden die Kulturen bei 24°C einen Tag lang bei Dunkelheit, dann 6 Tage bei Licht gehalten. Anschliessend wird die Protoplasten-haltige Agarose in Viertel geschnitten und in flüssiges Medium eingeführt. Die Protoplasten werden nun nach der "bead-type"-Kulturmethode kultiviert. In Kallusgeweben, welche durch Selektionierung des transformierten Materials mittels Kanamycin erhalten werden und in daraus regenerierten Pflanzen ist das NPT-II-Enzym (Aminoglykosidphosphotransferase) als Produkt des NPT-II-Gens vorhanden.

Mit der Elektroporation wird gegenüber der entsprechenden Methode ohne Elektroporation eine Steigerung der Transformationshäufigkeit um das 5- bis 10fache erreicht. Analoge Versuche mit Brassica rapa c.v. Just Right und Lolium multiflorum erbringen ebenfalls eine Steigerung der Transformationshäufigkeit in gleicher Grössenordnung.

Beispiel 6: Transformation von Zellen von Nicotiana tabacum durch Uebertragung des NPT-II-Gens unter Anwendung der Hitzeschockbehandlung

Protoplasten aus Blättern oder Zellkulturen von Nicotiana tabacum werden isoliert wie in den Beispielen 1 und 4 beschrieben und in ein osmotisches Medium, wie in den vorgängigen Beispielen beschrieben, überführt. Die Protoplastensuspension wird 5 Minuten lang bei einer Temperatur von 45°C gehalten, innerhalb 10 Sekunden mit Eis abgekühlt und anschliessend mit dem Plasmid pABDI versetzt wie in den Beispielen 1 und 4 beschrieben. Die Transformationshäufigkeit wird durch die Hitzeschockbehandlung gegenüber einer Transformation ohne diese Massnahme um einen Faktor von 10 oder grösser gesteigert.

Analoge Versuche mit den in den Beispielen 2 und 3 beschriebenen Protoplasten und Plasmiden ergeben ebenfalls eine Steigerung der Transformationshäufigkeit in gleicher Grössenordnung.

Beispiel 7: Transformation verschiedener Pflanzenzellen durch Uebertragung des NPT-II-Gens unter Zusammenführung von Protoplasten und Gen als erste Massnahme und anschliessender kombinierter Behandlung

Protoplasten der Pflanzen:
Nicotiana tabacum c.v. Petit Havanna SRI (A),
Brassica rapa c.v. Just Right (B) und
Lolium multiflorum (C)
werden isoliert und in osmotisches Medium überführt wie in Beispiel 5 beschrieben. Die Protoplastensuspensionen von A) und C) werden mit dem Plasmid pABDI (Beispiel 1a), diejenige von B) mit dem Plasmid pCaMV6km (Beispiel 2a) versetzt, wie in den Beispielen 1 bis 3 beschrieben, jedoch ohne gleichzeitige Behandlung mit Poläthylenglykol. Anschliessend werden die Protoplastensuspensionen einer Hitzeschockbehandlung gemäss Beispiel 6, dann einer Polyäthylenglykolbehandlung wie in den Beispielen 1 bis 3 beschrieben, und anschliessend der Elektroporation gemäss Beispiel 5 unterworfen. Die Transformationshäufigkeit bei diesem Vorgehen liegt im Bereich von $10^{-3}$ bis $10^{-2}$, wobei je nach Bedingungen 1 bis 2 % erreicht werden können. (Transformationshäufigkeit bei den Beispielen 1 bis 3 bei etwa $10^{-5}$). Ergebnisse im Bereich von $10^{-3}$ bis $10^{-2}$ werden auch erzielt, wenn nach dem Zusammenbringen von Protoplasten und Plasmiden die nachfolgenden Massnahmen der Hitzeschockbehandlung, der Polyäthylenglykolbehandlung und der Elektroporation in anderer Reihenfolge eingesetzt werden.

**Patentansprüche**

1. Verfahren zur direkten Transformation von Genen in pflanzliches Erbgut, dadurch gekennzeichnet, dass man
   a) ein lineares DNS-Molekül oder ein Plasmid, das ein Gen enthält, welches unter der Kontrolle von pflanzenwirksamen Expressionssignalen steht, in einem geeigneten Medium und unter Anwendung geeigneter Verfahrensmassnahmen, die die Protoplasten für die Aufnahme von DNS kompetent machen, mit pflanzlichen Protoplasten in Kontakt bringt,
   b) besagtes lineares DNS-Molekül bzw. Plasmid und die Protoplasten dort für einen Zeitraum belässt, der für die Aufnahme der DNS in die Protoplasten ausreicht, wobei
   c) das Gen stabil in das Pflanzengenom eingebaut sowie dort exprimiert und repliziert wird, mit der Massgabe, dass die einzuschleusende DNS keine für die Einschleusung oder Integration wesentlichen Anteile aus Ti-Plasmiden enthält.

2. Verfahren gemäss Anspruch 1, dadurch gekennzeichnet, dass man
   a) ein lineares DNS-Molekül oder ein Plasmid, das ein Gen enthält, welches unter der Kontrolle von pflanzenwirksamen Expressionssignalen steht, in einem geeigneten Medium und unter Anwendung geeigneter Verfahrensmassnahmen, die die Protoplasten für die Aufnahme von DNS Molekülen kompetent machen, mit pflanzlichen Protoplasten in Kontakt bringt,
   b) besagtes lineares DNS-Molekül bzw. Plasmid und die Protoplasten dort für einen Zeitraum belässt, der für die Aufnahme der DNS in die Protoplasten ausreicht, wobei
   c) das Gen stabil in das Pflanzengenom eingebaut sowie dort exprimiert und repliziert wird, mit der Massgabe, dass die einzuschleusende DNS keine DNS-Abschnitte aus der vir-Region oder den T-DNS Borderregionen eines Ti-Plasmids enthält.

3. Verfahren gemäss einem der Ansprüche 1 oder 2, dadurch gekennzeichnet, dass man ein osmotisch stabilisiertes Kulturmedium verwendet.

4. Verfahren gemäss einem der Ansprüche 1 oder 2, dadurch gekennzeichnet, dass man besagte einzuschleusende DNS und Protoplasten über einen Zeitraum von einigen Sekunden bis zu einigen Stunden miteinander in Kontakt bringt.

5. Verfahren gemäss Anspruch 1, dadurch gekennzeichnet, dass man für die Transformation eine genetische Konstruktion verwendet, die aus einem Strukturgen und pflanzenwirksamen Expressionssignalen besteht, welche von neutraler Träger-DNS flankiert werden.

6. Verfahren gemäss Anspruch 5, dadurch gekennzeichnet, dass das Strukturgen und die Expressionssignale homologen und/oder heterologen Ursprungs sind in Bezug auf das zu transformierende Pflanzengenom.

7. Verfahren gemäss Anspruch 5, dadurch gekennzeichnet, dass man eine genetische Konstruktion verwendet, deren Strukturteil pflanzlicher, tierischer, mikrobieller, viraler oder synthetischer Herkunft ist und deren Expressionssignale pflanzlicher, tierischer, viraler oder synthetischer Herkunft sind.

8. Verfahren gemäss Anspruch 7, dadurch gekennzeichnet, dass man Expressionssignale von Pflanzenviren verwendet.

9. Verfahren gemäss einem der Ansprüche 1 oder 2, dadurch gekennzeichnet, dass die Einschleusung der DNS in die Protoplasten mittels Polyäthylenglykolbehandlung durchgeführt wird.

10. Verfahren gemäss einem der Ansprüche 1 oder 2, dadurch gekennzeichnet, dass die Einschleusung der DNS in die Protoplasten mittels Hitzeschockbehandlung durchgeführt wird.

11. Verfahren gemäss einem der Ansprüche 1 oder 2, dadurch gekennzeichnet, dass die Einschleusung der DNS in die Protoplasten mittels Elektroporation durchgeführt wird.

12. Verfahren gemäss einem der Ansprüche 1 oder 2, dadurch gekennzeichnet, dass die Einschleusung der DNS in die Protoplasten mittels einer Kombination von zwei oder drei der Techniken Polyäthylenglykolbehandlung, Hitzeschockbehandlung und Elektroporation durchgeführt wird.

13. Verfahren gemäss einem der Ansprüche 1 oder 2, dadurch gekennzeichnet, dass die Einschleusung der DNS in die Protoplasten mittels einer Kombination von zwei oder drei der Techniken Polyäthylenglykolbehandlung, Hitzeschockbehandlung und Elektroporation, welche erst nach dem Einbringen von DNS und Protoplasten in eine Lösung zur Anwendung gelangen, durchgeführt wird.

14. Verfahren gemäss einem der Ansprüche 1 oder 2, dadurch gekennzeichnet, dass die Einschleusung der DNS in die Protoplasten durch Einbringen von DNS und Protoplasten in eine Lösung, anschliessende Hitzeschockbehandlung und darauffolgende Polyäthylenglykolbehandlung durchgeführt wird.

15. Verfahren gemäss einem der Ansprüche 1 oder 2, dadurch gekennzeichnet, dass die Einschleusung der DNS in die Protoplasten durch Einbringen von DNS und Protoplasten in eine Lösung, anschliessende Hitzeschockbehandlung, darauffolgende Polyäthylenglykolbehandlung und abschliessende Elektroporation durchgeführt wird.

16. Verfahren gemäss einem der Ansprüche 1 oder 2, dadurch gekennzeichnet, dass die Einschleusung der DNS in die Protoplasten in Gegenwart pflanzenverträglicher, zweiwertiger Kationen durchgeführt wird.

17. Verfahren gemäss einem der Ansprüche 1 oder 2, dadurch gekennzeichnet, dass es sich bei den Kationen um Magnesium- oder Calcium-Kationen handelt.

18. Verfahren gemäss einem der Ansprüche 1 oder 2, dadurch gekennzeichnet, dass die Einschleusung der DNS in die Protoplasten bei einem pH-Wert von 9 bis 10,5 durchgeführt wird.

19. Verfahren gemäss einem der Ansprüche 1 oder 2, dadurch gekennzeichnet, dass es sich um Protoplasten von Pflanzen ausgewählt aus der Gruppe bestehend aus den Familien Solanaceae, Cruciferae, Compositae, Liliaceae, Vitaceae, Chenopodiaceae, Rutaceae, Bromeliaceae, Rubiaceae, Theaceae, Musaceae oder Gramineae und der Ordnung Leguminosae handelt.

20. Verfahren gemäss Anspruch 19, dadurch gekennzeichnet, dass es sich um Protoplasten der Familien Solanaceae, Cruciferae und Gramineae handelt.

21. Verfahren gemäss einem der Ansprüche 1 oder 2 zur Transformation von Pflanzen, welche aus Protoplasten regenerierbar sind.

22. Verfahren gemäss einem der Ansprüche 1 oder 2 zur Transformation von Pflanzen ausgewählt aus der Gruppe bestehend aus Nicotiana tabacum, Nicotiana plumbaginifolia, Petunia hybrida, Hyoscyamus muticus und Brassica napus mittels Uebertragung des NPT-II-Gens, dadurch gekennzeichnet, dass man das NPT-II-Gen mit Promotor- und Terminationssignalen des CaMV-Gens VI versieht, dieses in das pCU8-Plasmid einbaut und das erhaltene chimäre Plasmid durch Polyäthylenglykolbehandlung in isolierte Protoplasten von Pflanzen der vorstehend genannten Gruppe überträgt.

23. Verfahren gemäss Anspruch 22 zur Transformation von Tabakpflanzen mittels Uebertragung des NPT-II-Gens, dadurch gekennzeichnet, dass man das NPT-II-Gen mit Promotor- und Terminationssignalen des CaMV-Gens VI versieht, dieses in das pUC8-Plasmid einbaut und das erhaltene chimäre Plasmid durch Polyäthylenglykol-Behandlung in isolierte Tabakprotoplasten überträgt.

24. Verfahren gemäss einem der Ansprüche 1 oder 2 zur Transformation von Pflanzen der Gattung Brassica mittels Uebertragung des NPT-II-Gens, dadurch gekennzeichnet, dass man das NPT-II-Gen mit Promotor- und Terminationssignalen des CaMV-Gens VI versieht, diese Konstruktion anstelle des CaMV-Gens VI in das CaMV-Genom einbaut und das erhaltene chimäre Plasmid durch Polyäthylenglykol-Behandlung in isolierte Brassicaprotoplasten überträgt.

25. Verfahren gemäss einem der Ansprüche 1 oder 2 zur Transformation von Pflanzen der Gattung Lolium mittels Uebertragung des NPT-II-Gens, dadurch gekennzeichnet, dass man das NPT-II-Gen mit Promotor- und Terminationssignalen des CaMV-Gens VI versieht, dieses in das pUC8-Plasmid einbaut und das erhaltene chimäre Plasmid durch Polyäthylenglykol-Behandlung in isolierte Loliumprotoplasten überträgt.

26. Verfahren zur Herstellung transgener Pflanzen, die homologe oder heterologe DNS in experimierbarer Form stabil in ihr Genom integriert enthalten, dadurch gekennzeichnet, dass man
   a) ein lineares DNS-Molekül oder Plasmid, das ein Gen enthält, welches unter der Kontrolle von pflanzenwirksamen Expressionssignalen steht, in einem geeigneten Medium und unter Anwendung geeigneter Verfahrensmassnahmen, die die Protoplasten für die Aufnahme von DNS kompetent machen, direkt in pflanzliche Protoplasten einschleust,
   b) besagtes lineares DNS-Molekül bzw. Plasmid und die Protoplasten dort für einen Zeitraum belässt, der für die Aufnahme der DNS in die Protoplasten ausreicht, wobei
c) das Gen stabil in das Pflanzengenom eingebaut sowie dort exprimiert und repliziert wird, mit der Massgabe, dass die einzuschleusende DNS keine für die Einschleusung oder Integration wesentlichen Anteile aus Ti-Plasmiden enthält, und
d) aus den zuvor transformierten Protoplasten transgene Pflanzen regeneriert, welche das eingeschleuste Gen stabil in ihr Genom eingebaut enthalten.

27. Verfahren gemäss Anspruch 26, dadurch gekennzeichnet, dass es sich bei besagten Anteilen aus Ti-Plasmiden um DNS-Abschnitte aus der vir-Region oder den T-DMS Borderregionen des Ti-Plasmids handelt.

**Claims**

1. A process for the direct transformation of genes in plant hereditary material, which comprises
   a) bringing a linear DNA molecule or a plasmid which contains a gene which is under the control of expression signals having effect in plants, in a suitable medium and using suitable procedures which render the protoplasts competent for the uptake of DNA, into contact with plant protoplasts,
   b) leaving said linear DNA molecule or plasmid and the protoplasts there for a period of time sufficient for the DNA to be taken up into the protoplasts, where
   c) the gene is incorporated into the plant genome in a stable manner and expressed and replicated there, with the proviso that the DNA to be incorporated does not contain any Ti plasmid portions essential to the incorporation or integration.

2. A process according to claim 1, which comprises
   a) bringing a linear DNA molecule or a plasmid which contains a gene which is under the control of expression signals having effect in plants, in a suitable medium and using suitable procedures which render the protoplasts competent for the uptake of DNA, into contact with plant protoplasts,
   b) leaving said linear DNA molecule or plasmid and the protoplasts there for a period of time sufficient for the DNA to be taken up into the protoplasts, where
   c) the gene is incorporated into the plant genome in a stable manner and expressed and replicated there, with the proviso that the DNA to be incorporated does not contain any DNA sections from the vir region or the carrier DNA border regions of a Ti plasmid.

3. A process according to one of claims 1 and 2, wherein an osmotically stabilised culture medium is used.

4. A process according to one of claims 1 and 2, wherein said DNA to be incorporated and protoplasts are brought into contact with one another for a period of time extending from several seconds up to several hours.

5. A process according to claim 1, which comprises using for the transformation a genetic construction consisting of a structural gene and expression signals, having effect in plants, which are flanked by neutral carrier DNA.

6. A process according to claim 5, wherein the structural gene and the expression signals are of homologous and/or heterologous origin in relation to the plant genome to be transformed.

7. A process according to claim 5, wherein a genetic construction is used whose structural part is of plant, animal, microbial, viral or synthetic origin and whose expression signals are of plant, animal or synthetic origin.

8. A process according to claim 7, wherein expression signals of plant viruses are used.

9. A process according to one of claims 1 and 2, wherein the incorporation of the DNA into the protoplasts is carried out by polyethylene glycol treatment.

10. A process according to one of claims 1 and 2, wherein the incorporation of the DNA into the protoplasts is carried out by heat shock treatment.

11. A process according to one of claims 1 and 2, wherein the incorporation of the DNA into the protoplasts is carried out by electroporation.

12. A process according to one of claims 1 and 2, wherein the incorporation of the DNA into the protoplasts is carried out by a combination of two or three of the techniques comprising polyethylene glycol treatment, heat shock treatment and electroporation.

13. A process according to one of claims 1 and 2, wherein the incorporation of the DNA into the protoplasts is carried out by a combination of two or three of the techniques comprising polyethylene glycol treatment, heat shock treatment and electroporation, which technique is employed only after introducing the DNA and the protoplasts into a solution.

14. A process according to one of claims 1 and 2, wherein the incorporation of the DNA into the protoplasts is carried out by introducing the DNA and the protoplasts into a solution, followed by heat shock treatment and subsequently polyethylene glycol treatment.

15. A process according to one of claims 1 and 2, wherein the incorporation of the DNA into the protoplasts is carried out by introducing the DNA and the protoplasts into a solution, followed by heat shock treatment, subsequently polyethylene glycol treatment and ultimately electroporation.

16. A process according to one of claims 1 and 2, wherein the incorporation of the DNA into the protoplasts is carried out in the presence of divalent cations which are tolerated by plants.

17. A process according to one of claims 1 and 2, wherein the cations are magnesium or calcium cations.

18. A process according to one of claims 1 and 2, wherein the incorporation of the DNA into the protoplasts is carried out at a pH of 9 to 10.5.

19. A process according to one of claims 1 and 2, wherein the protoplasts are protoplasts of plants selected from the group comprising the families Solanaceae, Cruciferae, Compositae, Liliaceae, Vitaceae, Chenopodiaceae, Rutaceae, Bromeliaceae, Rubiaceae, Theaceae, Musaceae or Gramineae or of the order Leguminosae.

20. A process according to claim 19, wherein the protoplasts are protoplasts of the families Solanaceae, Cruciferae and Gramineae.

21. A process according to one of claims 1 and 2 for the transformation of plants which can be regenerated from protoplasts.

22. A process according to one of claims 1 and 2 for the transformation of plants selected from the group consisting of Nicotiana tabacum, Nicotiana plumbaginifolia, Petunia hybrida, Hyoscyamus muticus and Brassica napus by transfer of the NPT II gene, which comprises providing the NPT II gene with promoter and termination signals of the CaMV gene VI, inserting said gene into the pCU8 plasmid, and transferring the resultant chimaeric plasmid by polyethylene glycol treatment into isolated protoplasts of plants of the aforementioned group.

23. A process according to claim 22 for the transformation of tobacco plants by transfer of the NPT II gene, which comprises providing the NPT II gene with promoter and termination signals of the CaMV gene VI, inserting said gene into the pUC8 plasmid and transferring the resultant chimaeric plasmid into isolated tobacco protoplasts by polethylene glycol treatment.

24. A process according to one of claims 1 and 2 for the transformation of plants of the genus Brassica by transfer of the NPT II gene, which comprises providing the NPT II gene with promoter and termination signals of the CaMV gene VI, inserting this construction instead of the CaMV gene VI into the CaMV genome, and transferring the resultant chimaeric plasmid into isolated Brassica protoplasts by polyethylene glycol treatment.

25. A process according to one of claims 1 and 2 for the transformation of plants of the genus Lolium by transfer of the NPT II gene, which comprises providing the NPT II gene with promoter and termination signals of the CaMV gene VI, inserting said gene into the pUC8 plasmid, and transferring the resultant chimaeric plasmid into isolated Lolium protoplasts by polyethylene glycol treatment.

26. A process for the production of transgenic plants which contain homologous or heterologous DNA in an expressible form integrated into their genome in a stable manner, which comprises

a) incorporating a linear DNA molecule or a plasmid which contains a gene which is under the control of expression signals having effect in plants, in a suitable medium and using suitable procedures which render the protoplasts competent for the uptake of DNA, directly into plant protoplasts,

b) leaving said linear DNA molecule or plasmid and the protoplasts there for a period of time sufficient for the DNA to be taken up into the protoplasts, where

c) the gene is incorporated into the plant genome in a stable manner and expressed and replicated there, with the proviso that the DNA to be incorporated does not contain any Ti plasmid portions essential to the incorporation or integration, and

d) regenerating transgenic plants from the previously transformed protoplasts, which contain the incorporated gene inserted into their genome in a stable manner.

27. A process according to claim 26, wherein said Ti plasmid portions are DNA sections from the vir region or the carrier DNA border regions of the Ti plasmid.

**Revendications**

1. Procédé pour la transformation directe de gènes dans un génotype végétal, caractérisé en ce qu'on

   a) met en contact avec des protoplastes végétaux une molécule d'ADN linéaire ou un plasmide, qui contient un gène se trouvant sous le contrôle de signaux d'expression actifs sur les plantes, dans un milieu approprié et en utilisant des conditions opératoires appropriées qui rendent les protoplastes compétents pour la fixation de l'ADN,

   b) laisse ladite molécule d'ADN linéaire ou ledit plasmide et les protoplastes dans ces conditions pendant une durée suffisante pour la fixation de l'ADN dans les protoplastes, tandis que

   c) le gène est introduit de manière stable dans le génome des plantes et y est répliqué, avec pour condition que l'ADN à introduire ne contienne aucune fraction de plasmides Ti essentielle pour l'introduction ou intégration.

2. Procédé selon la revendication 1, caractérisé en ce qu'on

   a) met en contact avec des protoplastes végétaux une molécule d'ADN linéaire ou un plasmide, qui contient un gène se trouvant sous le contrôle de signaux d'expression actifs sur les plantes, dans un milieu approprié et en utilisant des conditions opératoires appropriées qui rendent les protoplastes compétents pour la fixation de molécules d'ADN,

   b) laisse ladite molécule d'ADN linéaire ou ledit plasmide et les protoplastes dans ces conditions pendant une durée suffisante pour la fixation de l'ADN dans les protoplastes, tandis que

   c) le gène est introduit de manière stable dans le génome des plantes et y est répliqué, avec pour condition que l'ADN à introduire ne contienne aucune fraction de la région vir ou des régions extrêmes d'ADN T d'un plasmide Ti.

3. Procédé selon l'une des revendications 1 ou 2, caractérisé en ce qu'on utilise un milieu de culture stable osmotiquement.

4. Procédé selon l'une des revendications 1 ou 2, caractérisé en ce qu'on met lesdits ADN à introduire et protoplastes en contact entre eux pendant une durée de quelques secondes à quelques heures.

5. Procédé selon la revendication 1, caractérisé en ce qu'on utilise pour la transformation une construction génétique consistant en un gène de structure et des signaux d'expression actifs sur les plantes, qui sont accompagnés d'ADN support neutre.

6. Procédé selon la revendication 5, caractérisé en ce que le gène de structure et les signaux d'expression sont d'origine homologue et/ou hétérologue par rapport au génome des plantes à transformer.

7. Procédé selon la revendication 7, caractérisé en ce qu'on utilise une construction génétique dont la partie structurale est d'origine végétale, animale, microbienne, virale ou synthétique et dont les signaux d'expression sont d'origine végétale, animale, virale ou synthétique.

8. Procédé selon la revendication 7, caractérisé en ce qu'on utilise des signaux d'expression d'origine végétale.

9. Procédé selon l'une des revendications 1 ou 2, caractérisé en ce qu'on effectue l'introduction de l'ADN dans les protoplastes au moyen d'un traitement au polyéthylèneglycol.

10. Procédé selon l'une des revendications 1 ou 2, caractérisé en ce qu'on effectue l'introduction de l'ADN dans les protoplastes au moyen d'un traitement par choc thermique.

11. Procédé selon l'une des revendications 1 ou 2, caractérisé en ce qu'on effectue l'introduction de l'ADN dans les protoplastes au moyen d'une électroporation.

12. Procédé selon l'une des revendications 1 ou 2, caractérisé en ce qu'on effectue l'introduction de l'ADN dans les protoplastes au moyen d'une combinaison de deux ou trois des techniques que sont le traitement au polyéthylèneglycol, le traitement par choc thermique et l'électroporation.

13. Procédé selon l'une des revendications 1 ou 2, caractérisé en ce qu'on effectue l'introduction de l'ADN dans les protoplastes au moyen d'une combinaison de deux ou trois des techniques que sont le traitement au polyéthylèneglycol, le traitement par choc thermique et l'électroporation, qui ne sont employées qu'après la mise en solution de l'ADN et des protoplastes.

14. Procédé selon l'une des revendications 1 ou 2, caractérisé en ce qu'on effectue l'introduction de l'ADN dans les protoplastes par mise en solution de l'ADN et des protoplastes, puis traitement par choc thermique et traitement subséquent au polyéthylèneglycol.

15. Procédé selon l'une des revendications 1 ou 2, caractérisé en ce qu'on effectue l'introduction de l'ADN dans les protoplastes par mise en solution de l'ADN et des protoplastes, puis traitement par choc thermique, suivi d'un traitement au polyéthylèneglycol et enfin électroporation.

16. Procédé selon l'une des revendications 1 ou 2, caractérisé en ce qu'on effectue l'introduction de l'ADN dans les protoplastes en présence de cations bivalents compatibles avec les plantes.

17. Procédé selon l'une des revendications 1 ou 2, caractérisé en ce qu'il s'agit de cations de magnésium ou de calcium.

18. Procédé selon l'une des revendications 1 ou 2, caractérisé en ce qu'on effectue l'introduction de l'ADN dans les protoplastes à un pH de 9 à 10,5.

19. Procédé selon l'une des revendications 1 ou 2, caractérisé en ce qu'il s'agit de protoplastes de plantes choisies parmi les familles Solanaceae, Cruciferae, Compositae, Liliaceae, Vitaceae, Chenopodiceae, Rutaceae, Bromeliaceae, Rubiaceae, Theaceae, Musaceae ou Gramineae et l'ordre des Leguminosae.

20. Procédé selon la revendication 19, caractérisé en ce qu'il s'agit de protoplastes des familles des Solanaceae, Cruciferae et Gramineae.

21. Procédé selon l'une des revendications 1 ou 2 pour la transformation de plantes, qui peuvent être régénérées à partir de protoplastes.

22. Procédé selon l'une des revendications 1 ou 2 pour la transformation de plantes choisies dans le groupe comprenant Nicotiana tabacum, Nicotiana plumbaginifolia, Petunia hybrida, Hyoscyamus muticus et Brassica napus au moyen d'un transfert du gène de NPT-II, caractérisé en ce qu'on munit le gène NPT-II de signaux de promotion et de terminaison du gène VI de CaMV, on incorpore celui-ci dans le plasmide pCU8 et on transfère le plasmide chimérique obtenu par traitement au polyéthylèneglycol dans des protoplastes isolés de plantes du groupe susmentionné.

23. Procédé selon la revendication 22 pour la transformation de plants de tabac au moyen d'un transfert du gène de NPT-II, caractérisé en ce qu'on munit le gène NPT-II de signaux de promotion et de terminaison du gène VI de CaMV, on incorpore celui-ci dans le plasmide pUC8 et on transfère le plasmide chimérique obtenu par traitement au polyéthylèneglycol dans des protoplastes de tabac isolés.

24. Procédé selon l'une des revendications 1 ou 2 pour la transformation de plantes du genre Brassica au moyen d'un transfert du gène NPT-II, caractérisé en ce qu'on munit le gène NPT-II de signaux de promotion et de terminaison du gène VI de CaMV, on incorpore cette construction à la place du gène VI de CaMV dans le génome de CaMV et on transfère le plasmide chimérique obtenu par traitement au polyéthylèneglycol dans des protoplastes de Brassica isolés.

25. Procédé selon l'une des revendications 1 ou 2 pour la transformation de plantes du genre Lolium au moyen d'un transfert du gène NPT-II, caractérisé en ce qu'on munit le gène NPT-II de signaux de promotion et de terminaison du gène VI de CaMV, on incorpore celui-ci dans le plasmide pUC8 et on transfère le plasmide chimérique obtenu par traitement au polyéthylèneglycol dans des protoplastes de Lolium isolés.

26. Procédé pour la préparation de plantes transgéniques, qui contiennent de l'ADN homologue ou hétérologue, intégré dans leur génome, sous forme exprimable, stable, caractérisé en ce qu'on

    a) introduit directement dans des protoplastes de plantes une molécule d'ADN linéaire ou un plasmide, qui contient un gène se trouvant sous le contrôle de signaux d'expression actifs sur les plantes, dans un milieu approprié et en utilisant des conditions opératoires appropriées qui rendent les protoplastes compétents pour la fixation de l'ADN, b) laisse ladite molécule d'ADN linéaire ou ledit plasmide et les protoplastes dans ces conditions pendant une durée suffisante pour la fixation de l'ADN dans les protoplastes, tandis que

c) le gène est introduit de manière stable dans le génome des plantes et y est répliqué, avec pour condition que l' ADN à introduire ne contienne aucune fraction de plasmides Ti essentielles pour l'introduction ou intégration, et

d) régénère à partir des protoplastes préalablement transformés des plantes transgéniques, qui contiennent le gène inséré introduit de manière stable dans leur génome.

27. Procédé selon la revendication 26, caractérisé en ce qu'il s'agit pour les fractions susidtes de plasmides Ti de fragments d'ADN de la région vir ou des régions extrêmes d'ADN T du plasmide Ti.

**Fig. 1**

GEN VI REGION DES CAULIFLOWER-MOSAIK-VIRUS

HERSTELLUNG DER PLASMIDE pABD I UND pABD II

**Fig. 2**  HERSTELLUNG DES PLASMIDS pCaMV6km